# EUROPEAN PATENT APPLICATION

(11) **EP 4 424 259 A1**
(43) Date of publication of application: **04.09.2024**
(21) Application number: 22886135.7
(22) Date of filing: 28.10.2022
(51) Int. Cl.: A61B 18/14, A61B 17/32

(54) **CUTTING CONTROL METHOD AND APPARATUS APPLIED TO ULTRASONIC SCALPEL, AND STORAGE MEDIUM**

(30) Priority: 28.10.2021 CN 202111259038
(71) Applicant: B. J. Zh. F. Panther Medical Equipment Co., Ltd., Beijing 102200 (CN)
(72) Inventor: LIU, Qing, Beijing 102200 (CN); YAO, Yinfeng, Beijing 102200 (CN); GU, Linfei, Beijing 102200 (CN); ZHOU, Qingjie, Beijing 102200 (CN)
(74) Representative: Huang, Liwei
(86) International application number: PCT/CN2022/128318
(87) International publication number: WO 2023/072260

(57) **Abstract**

A cutting control method, device and storage medium applied to an ultrasonic knife. The method comprises: detecting a present active power of the ultrasonic knife (S101); and when the present active power is greater than a predetermined power critical value, and in the case that a duration of the present active power greater than the power critical value reaches a first predetermined time, a rated operating current of the ultrasonic knife is gradually lowered in a stepwise manner according to a predetermined time interval (S102). The control method and device accurately identify whether the ultrasonic knife enters the cutting end by detecting the active power of the ultrasonic knife, so that the operating current of the ultrasonic knife can be adjusted in time to lower the energy output of the ultrasonic knife when it enters the cutting end, that is, the cutting tissue becomes less, so as to effectively prevent the thermal damage of the tissue.

## Description

### TECHNICAL FIELD

The present application relates to the technical field of ultrasonic knife, in particular to a cutting control method, device and storage medium applied to an ultrasonic knife.

### BACKGROUND

An ultrasonic cutting and hemostatic knife, referred to as an ultrasonic knife, is a new medical device that has been used in surgical operations abroad since the late 1980s. It is generally used in thoracoabdominal surgery, because it can complete cutting and hemostasis at the same time, it is deeply favored by doctors and has become an important tool in surgery.

The ultrasonic knife generates heat through high-frequency vibration in the process of work, and the heat energy generated by this friction can coagulate and stop bleeding while cutting tissue. However, due to the limitation of the control algorithm and hardware, the existing front ultrasonic knife is generally controlled by AD sampling and averaging method, so the energy output cannot be adjusted in time in the second half of tissue cutting, and less tissue is cut at the cutting end, but the energy output of the ultrasonic knife is high, which will cause thermal damage of the tissue, and the incision is prone to yellowing or even blackening etc., that is, it will cause lateral thermal damage during cutting the tissue; the consequence of excessive cautery is carbonization of the incision. There is a possibility of shedding and secondary bleeding in the later stage.

Therefore, there is an urgent need for a cutting control method to solve the problem of tissue thermal damage caused by the ultrasonic knife at the cutting end.

### SUMMARY

In view of this, the present invention provides a cutting control method, device and storage medium applied to the ultrasonic knife, the main purpose of which is to solve the problem that the present ultrasonic knife will cause thermal damage to the cutting tissue at the cutting end.

In order to solve the above problem, the present application provides a cutting control method applied to an ultrasonic knife, comprising:
detecting a present active power of the ultrasonic knife;
when the present active power is greater than a predetermined power critical value, and in the case that a duration of the present active power greater than the power critical value reaches a first predetermined time, a rated operating current of the ultrasonic knife is gradually lowered in a stepwise manner according to a predetermined time interval.

Optionally, before detecting the present active power of the ultrasonic knife, the method further comprises determining the predetermined power critical value, specifically comprising:
determining an operating current range based on the rated operating current at an initial working moment of the ultrasonic knife;
detecting in real-time and obtaining a present operating current;
when the present operating current meets the operating current range, and in the case that a duration of the present operating current meeting the operating current range reaches a second predetermined time, detecting an active power at each moment within a predetermined time period;
a power critical value is determined based on the active powers.

Optionally, an operating current of the ultrasonic knife is gradually lowered in a stepwise manner according to the predetermined time interval, specifically comprising:
calculating and obtaining a target rated operating current based on a predetermined current ratio and a present rated operating current according to a predetermined time interval;
adjusting the present rated operating current of the ultrasonic knife to the target rated operating current.

Optionally, before adjusting the present rated operating current of the ultrasonic knife to the target rated operating current, the method further comprising:
judging whether a target rated current is less than a preset minimum rated current;
in the case that the target rated current is less than the minimum rated current, the present rated operating current of the ultrasonic knife is adjusted to the minimum rated current.

Optionally, the detecting in real-time and obtaining the present operating current, specifically comprising:
collecting a feedback signal output by an ultrasonic driving module in real-time to obtain sampling data;
processing the sampling data to obtain electrical parameters;
the present operating current is obtained based on the electrical parameters.

To solve the above technical problem, the present application provides a cutting control device applied to an ultrasonic knife, it comprises:
a detection module, is used to detect a present active power of the ultrasonic knife;
an adjustment module, is used to gradually lower in a stepwise manner a rated operating current of the ultrasonic knife according to a predetermined time interval when the present active power is greater than a predetermined power critical value, and in the case that a duration of the present active power greater than the power critical value reaches a first predetermined time.

Optionally, the cutting control device applied to an ultrasonic knife further comprises a determination module used to determine the predetermined power critical value, the determination module is specifically used to:
determining an operating current range based on the rated operating current at an initial working moment of the ultrasonic knife;
detecting in real-time and obtaining a present operating current;
when the present operating current meets the operating current range, and in the case that a duration of the present operating current meeting the operating current range reaches a second predetermined time, detecting an active power at each moment within a predetermined time period;
the power critical value is determined based on the active powers.

Optionally, the adjustment module is specially used to:
calculate and obtain a target rated operating current based on a predetermined current ratio and a present rated operating current according to a predetermined time interval;
adjust the present rated operating current of the ultrasonic knife to the target rated operating current.

Optionally, the cutting control device applied to an ultrasonic knife further comprises a judgment module, the judgement module is specially used to: judging whether the target rated current is less than the preset minimum rated current;
the judgment module is specially used to: in the case that the target rated current is less than the minimum rated current, the present rated operating current of the ultrasonic knife is adjusted to the minimum rated current.

To solve the above problem, the present application provides a storage medium, the storage medium stores a computer program, the computer program realizes the steps applied to the cutting control method of the ultrasonic knife of any of the above items when it is executed by a processor.

This present application detects the active power of the ultrasonic knife to accurately identify whether the ultrasonic knife has entered the cutting end, so that when it enters the cutting end, that is, when the cutting tissue becomes less, the operating current of the ultrasonic knife can be adjusted in time to lower the energy output of the ultrasonic knife, so as to effectively prevent tissue thermal damage.

The above description is only an overview of the technical solution of the present invention. In order to better understand the technical means of the present invention, it can be implemented according to the contents of the description, and in order to make the above and other purposes, features and advantages in the present invention more obvious and understandable, the specific embodiments of the present invention are enumerated below.

### BRIEF DESCRIPTION OF THE DRAWINGS

Various other advantages and benefits will become apparent to those of ordinary skill in the art upon reading the following detailed description of the preferred embodiment. The attached drawings are only for the purpose of illustrating a preferred embodiment and are not to be considered as limiting the invention. Furthermore, the same reference symbols are used to designate the same components throughout the attached drawings. In the attached drawings:
Fig. 1a is the voltage curve of the ultrasonic knife in the cutting process;
Fig. 1b is the current curve of the ultrasonic knife in the cutting process;
Fig. 1c is the load curve of the ultrasonic knife in the cutting process;
Fig. 2 is a flow chart of a cutting control method applied to an ultrasonic knife according to an embodiment of the present application;
Fig. 3 is the working principle diagram of the ultrasonic knife in the embodiment of the present application;
Fig. 4 is a flow chart of a cutting control method applied to an ultrasonic knife in another embodiment of the present application.

### DETAILED DESCRIPTION

Various aspects and features of the present application are described herein with reference to the accompanying drawings.

It should be understood that various modifications may be made to the embodiments applied for herein. Accordingly, the above description should not be viewed as limiting, but only as exemplifications of embodiments. Those skilled in the art will envision other modifications within the scope and spirit of the application.

The accompanying drawings, which are incorporated in and constitute a part of this specification, illustrate embodiments of the present application and, together with the general description of the application given above and the detailed description of the embodiments given below, serve to explain the principle of the present application.

These and other characteristics of the present application will become apparent from the following description of preferred forms of embodiment given as non-limiting examples with reference to the accompanying drawings.

It should also be understood that, while the application has been described with reference to a few specific examples, those skilled in the art will be able to implement certain other equivalents of the application.

The above and other aspects, features and advantages of the present application will become more apparent in view of the following detailed description when taken in conjunction with the accompanying drawings.

Specific embodiments of the present application are hereinafter described with reference to the accompanying drawings; however, it should be understood that the applied embodiments are merely examples of the present application, which can be implemented in various ways. Well-known and/or repetitive functions and constructions are not described in detail to avoid obscuring the application with unnecessary or redundant detail. Therefore, specific structural and functional details disclosed herein are not intended to be limiting, but merely as a basis for the claims and as a representative basis for teaching one skilled in the art to variously employ the present application in virtually any suitable detailed structure.

This specification may use the phrases "in one embodiment," "in another embodiment," "in yet another embodiment," or "in other embodiments," which may refer to the same or one or more of the different embodiments.

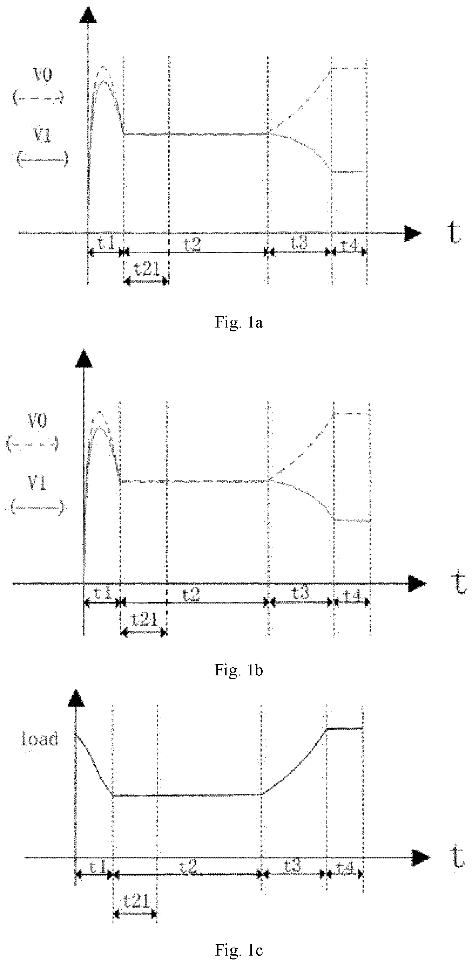

The embodiment of the present application provides a cutting control method applied to the ultrasonic knife, as shown in Fig. 1, in a complete cutting process of the ultrasonic knife, the voltage V0 curve can be shown as the dotted line in Fig. 1a, and the current 10 curve can be shown as the dotted line in Fig. 1b, the load curve may be as shown in Fig. 1c. It can be seen from Fig. 1a, Fig. 1b and Fig. 1c that a complete cutting can be divided into four periods from t1 to t4.

11: start the cutting stage, at this stage, the knife head changes from static to vibration. It needs to overcome a certain resistance. The load is relatively high, but the time is relatively short, and generally it will not affect the cutting effect.

t2: at this balanced cutting stage, the load in this stage is in a balanced state, and it is the main stage of cutting and hemostasis.

t3: at the cutting end, because this stage is about to be cut off, the load will increase, and at the same time, the tissue will become less, and the heat will be released intensively, this is the main stage that causes thermal damage.

t4: at this stage of tissue cutting completion, the actual tissue cutting is completed at this stage, but the working order has not been revoked. The knife head continues to vibrate and cut the cutting pad. The time of this stage depends on the reaction speed of the person. This stage will also cause some damage to the cutting pad.

Therefore, this embodiment provides a cutting control method of the ultrasonic knife by accurately identifying whether it has entered the cutting end, that is, judging whether it has entered the t3 stage, thereby timely controlling and reducing the energy output of the ultrasonic knife when entering the t3 stage, thereby lowering tissue thermal damage. Specifically, the voltage V1 curve after adopting the cutting control method in the present application can be shown as the solid line in Fig. 1a, and the current I1 curve can be shown as the solid line in Fig. 1b.

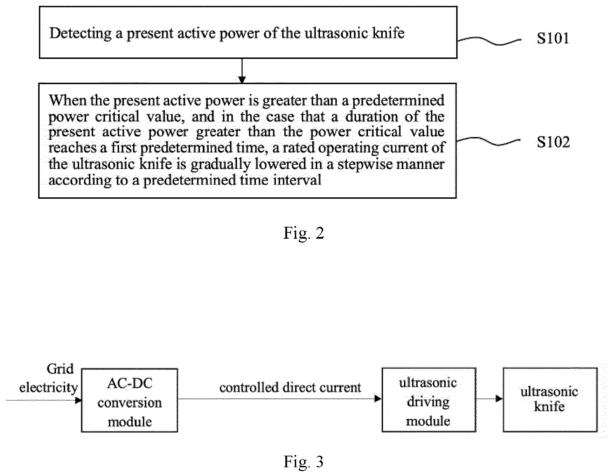

Specifically, as shown in Fig. 2, the cutting control method applied to the ultrasonic knife in this embodiment specifically comprises the following steps:
Step S101, detecting a present active power of the ultrasonic knife;
During the specific implementation at this step, before the present active power is detected, the power critical value may be determined in advance. The process of determining the power critical value is: based on the rated operating current at the initial working time of the ultrasonic knife, determine the operating current range; detect in real time and obtain the present operating current; when the present operating current meets the operating current range and in the case that the present operating current meets a duration of the operating current range reaches a second predetermined time, detect active power at each moment within the predetermined time period; determine the power critical value based on each active power. In this present application, the corresponding rated operating current Is is determined according to the working gear when the ultrasonic knife starts cutting, and then the operating current range is determined as (I_{S}×(1-X₀%), I_{S}×(1+X₀%)), wherein the value range of X₀ is 0.1-10, which can be adjusted according to actual requirements, so that when the subsequent real-time detected operating current Is' meets the above-mentioned operating current range, and when the met duration reaches the second predetermined time Ts, it means that the ultrasonic knife enters the balanced cutting stage (t2 stage) from the starting cutting stage (t1 stage). Therefore, the average active power Ps of the balanced cutting stage can be calculated by detecting the instantaneous power at each moment at the balanced cutting stage, and then the power critical value P_{S} × (1+X₁%) obtained by calculation based on average active power Ps, which lays the foundation for the subsequent real-time detection of the present active power and the judgment of the present active power based on the power critical value. Wherein, the value range of X₁ is 5-20, which can be adjusted according to actual requirements. At this step, the set second predetermined time Ts is less than or equal to the time of stage t2, which can be specifically set according to actual experience, for example, the value range of the second predetermined time Ts is 0.2s-1.5s, etc.

Step S102, in the case that the present active power is greater than a predetermined power critical value, and a duration of the present active power greater than the power critical value reaches the first predetermined time, a rated operating current of the ultrasonic knife is gradually lowered in a stepwise manner according to a predetermined time interval.

In the specific implementation process of this step, after the power critical value is determined, the present active power detected in real time can be judged based on the power critical value. In the case that determining the present active power is greater than the predetermined power critical value, and the duration of the present active power greater than the power critical value reaches the first predetermined time Tq, it is determined that the ultrasonic knife enters the cutting end (t3 stage) from the balanced cutting stage (t2 stage). At this time, it can be according to the predetermined time interval △T, the rated operating current of the ultrasonic knife is gradually lowered in a stepwise manner, so as to control and reduce the energy output of the ultrasonic knife, and avoid the problem of thermal damage to the cutting components. The above-mentioned first predetermined time in this embodiment can be preset according to actual requirement, for example, the first predetermined time Tq can be set as 50ms, 60ms, 70ms, 80ms, 100ms, 120ms or 150ms, etc., the range of its value is 40ms-160ms. In this embodiment, it is possible to accurately identify whether the ultrasonic knife has entered the cutting end by detecting the active power of the ultrasonic knife, so that when the ultrasonic knife enters the cutting end, that is, when the cutting tissue becomes less, the operating current of the ultrasonic knife can be adjusted in time, to lower the energy output of the ultrasonic knife, thereby effectively preventing thermal damage of the cutting tissue.

In the specific implementation process of this embodiment, the operating current of the ultrasonic knife is gradually lowered in a stepwise manner according to a predetermined time interval, specifically comprises: calculating according to a predetermined time interval, based on a predetermined current ratio and the present rated operating current to obtain a target rated operating current; adjusting the present rated operating current of the ultrasonic knife to the target rated operating current. For example, the predetermined time interval is 10ms, the predetermined current ratio is 0.9, and the present rated operating current is Is, then the target rated operating current I can be calculated at every 10ms, I=0.9*Is, and then the present rated operating current of the ultrasonic knife is adjusted to the target rated operating current. In this embodiment, in order to prevent the operating current after the adjustment of the ultrasonic knife from being less than the minimum rated current of the normal operation of the ultrasonic knife, after calculating and obtaining the target rated operating current, the target rated operating current can be compared with the minimum rated current. When the target rated operating current is greater than the minimum rated current, the present rated operating current can be directly adjusted to the target rated operating current; when the target rated operating current is less than the minimum rated current, the present rated operating current of the ultrasonic knife is adjusted to the minimum rated current. During the specific implementation of this embodiment, several working gears can be preset for the ultrasonic knife according to the order of operating current from large to small. In this way, after the present rated operating current is determined, the working gear corresponding to the present rated operating current can be determined, and then it can be gradually adjusted to each working gear after the present working gear according to the predetermined time interval until it is adjusted to the lowest working gear.

In this embodiment, the working process of the ultrasonic knife can be shown in Figure 3: the AC-DC conversion module converts the alternating current AC input from the grid into a direct current DC, and outputs it to the ultrasonic driving module, and the ultrasonic driving module converts the direct current into the ultrasonic driving signal AC required by the ultrasonic knife (that is, converts into a specific frequency alternating current), and finally outputs the driving signal to the ultrasonic knife through the interface of the ultrasonic knife, and the piezoelectric ceramic chip of the ultrasonic knife driving handle converts the driving signal into the vibration of the ultrasonic knife. Therefore, when the present operating current is obtained by detection, the feedback signal output by the ultrasonic driving module can be collected in real time to obtain sampled data; the sampled data is processed to obtain electrical parameters, such as Fourier transform processing is performed on the sampled data to obtain electrical parameters such as harmonics, phase, voltage and current, etc.; the present operating current is obtained based on the electrical parameters, that is, the present actual operating current is obtained.

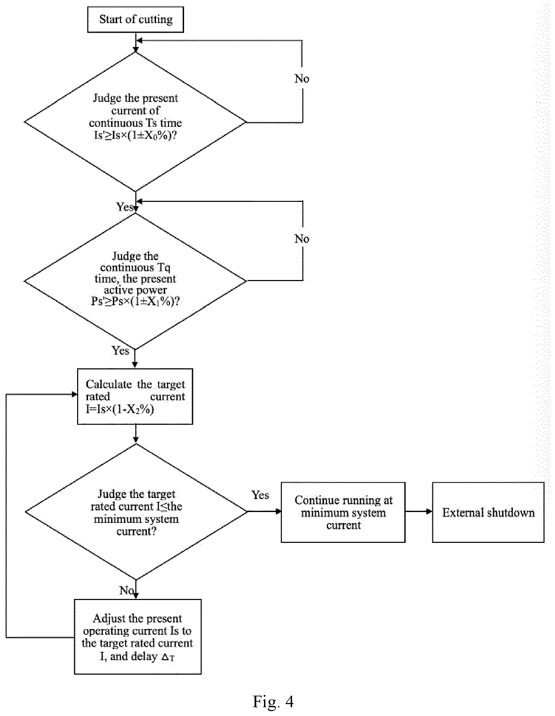

On the basis of the above embodiments, in order to further explain the solutions in the present application, the following descriptions will be made in conjunction with specific application scenarios. As shown in Fig. 4, the ultrasonic knife monitors the current in real time after the cutting starts; assuming that the present system working gear requires that a current is Is, when the real-time/present current Is' is greater than or equal to Is×(1±X₀%) at the continuing second predetermined time, that is, Ts time, determines that the system enters t2 (balanced cutting stage), and calculates the average active power in this time period, which is recorded as Ps. Specifically, the active power value of a predetermined time period at the balanced cutting stage can be calculated, and the predetermined time period can be set according to actual experience.

Then the cutting continues, and the present working power of the ultrasonic knife is continuously monitored. If the active power Ps' of the system is greater than Ps×(1+X₁%) within the continuous Tq time, then it is determined that the ultrasonic knife system enters t3 (cutting end). Adjusting the system working gear current to the target rated work I at every △_{T} interval until the system working gear current is adjusted to the system working minimum gear current. Among them, I=Is×(1-X₂%), X₂ and ΔT can be set according to actual requirement, such as the number of times to adjust the current down to the minimum gear working current is n, then the time of n×△T should cover time period t3 of the tissue cutting process.

The system continues to work, and the operating current will be lowered to the minimum gear current of the system working, which will not cause too much damage to the cutting pad.

Among them, Is is the working current of the present gear. Specifically, the ultrasonic knife can be divided into 5 gears, and each gearl has a preset rated operating current.

During the specific implementation of this embodiment, X₀ may take any value between 5-10. Ts can be 0.8-1.5 seconds according to the cutting time of blood vessels in experience. X₁ can be any value between 10-20. The possible value of Tq is 100ms. The possible value of X₂ is 5.

In this embodiment, by detecting the active power of the ultrasonic knife to accurately identify whether the ultrasonic knife has entered the cutting end, so that when the ultrasonic knife enters the cutting end, that is, when the cutting tissue becomes less, the operating current of the ultrasonic knife is adjusted in time to lower the energy output of the ultrasonic knife, thereby effectively preventing thermal damage of the tissue.

Another embodiment of the present application provides a cutting control device applied to an ultrasonic knife, including:
a detection module, is used to detect the present active power of the ultrasonic knife;
an adjustment module, is used to when the present active power is greater than a predetermined power critical value, and the duration of the present active power greater than the power critical value reaches a first predetermined time, according to a predetermined time interval, the rated operating current of the ultrasonic knife is gradually lowered in a stepwise manner.

Specifically, the cutting control device applied to the ultrasonic knife in this embodiment further includes a determination module for determining the predetermined power critical value, and the determination module is specifically used to: based on the rated operating current of the initial working time of the ultrasonic knife, determine the operating current range; the present operating current is obtained in real-time detection; when the present operating current meets the operating current range, and in the case that the duration of the present operating current meets the operating current range reaches the second predetermined time , detecting active power at each moment within a predetermined time period; determining the power critical value based on the active powers.

Specifically, the adjustment module is specifically used to: calculate and obtain the target rated operating current based on the predetermined current ratio and the present rated operating current according to a predetermined time interval; adjust the present rated operating current of the ultrasonic knife to the target rated operating current.

The cutting control device applied to the ultrasonic knife in this embodiment further includes a judgment module, the judgment module is specifically used to: judge whether the target rated current is less than a preset minimum rated current; the adjustment module is specifically used to: in the case that the target rated current is less than the minimum rated current, the present rated operating current of the ultrasonic knife is adjusted to the minimum rated current.

This application detects the active power of the ultrasonic knife to accurately identify whether the ultrasonic knife has entered the cutting end, so that when it enters the cutting end, that is, when the cutting tissue becomes less, the operating current of the ultrasonic knife can be adjusted in time, to lower the energy output of the ultrasonic knife, so as to effectively prevent tissue thermal damage.

Another embodiment of the present application provides a storage medium, the storage medium stores a computer program, and when the computer program is executed by a processor, the following method steps are implemented:
Step 1. detecting the present active power of the ultrasonic knife;
Step 2. when the present active power is greater than the predetermined power critical value, and in the case that the duration of the present active power greater than the power critical value reaches the first predetermined time, the rated operating current of the ultrasonic knife is gradually lowered in a stepwise manner according to a predetermined time interval.

The specific implementation process of the above method steps can be referenced any of the above embodiments of the cutting control method applied to the ultrasonic knife, and this embodiment will not be repeated here.

This application detects the active power of the ultrasonic knife to accurately identify whether the ultrasonic knife has entered the cutting end, so that when it enters the cutting end, that is, when the cutting tissue becomes less, the operating current of the ultrasonic knife can be adjusted in time, to lower the energy output of the ultrasonic knife, so as to effectively prevent tissue thermal damage.

The above embodiments are only exemplary embodiments of the present application, and are not intended to limit the present application, and the protection scope of the present application is defined by the claims. Those skilled in the art may make various modifications or equivalent replacements to the present application within the spirit and protection scope of the present application, and such modifications or equivalent replacements shall also be deemed to fall within the protection scope of the present application.

## Claims

1. A cutting control method applied to an ultrasonic knife, **characterized in** comprising:
detecting a present active power of the ultrasonic knife;
when the present active power is greater than a predetermined power critical value, and in the case that a duration of the present active power greater than the power critical value reaches a first predetermined time, a rated operating current of the ultrasonic knife is gradually lowered in a stepwise manner according to a predetermined time interval.

2. The method according to claim 1, **characterized in that**, before detecting the present active power of the ultrasonic knife, the method further comprises determining the predetermined power critical value, specifically comprising:
determining an operating current range based on the rated operating current at an initial working moment of the ultrasonic knife;
detecting in real-time and obtaining a present operating current;
when the present operating current meets the operating current range, and in the case that a duration of the present operating current meeting the operating current range reaches a second predetermined time, detecting an active power at each moment within a predetermined time period;
a power critical value is determined based on the active powers.

3. The method according to claim 1, **characterized in that**, the step that an operating current of the ultrasonic knife is gradually lowered in a stepwise manner according to the predetermined time interval specifically comprises:
according to a predetermined time interval, calculating and obtaining a target rated operating current based on a predetermined current ratio and a present rated operating current;
adjusting the present rated operating current of the ultrasonic knife to the target rated operating current.

4. The method according to claim 3, **characterized in that**, before adjusting the present rated operating current of the ultrasonic knife to the target rated operating current, the method further comprising:
judging whether a target rated current is less than a preset minimum rated current;
in the case that the target rated current is less than the minimum rated current, the present rated operating current of the ultrasonic knife is adjusted to the minimum rated current.

5. The method according to claim 2, **characterized in that**, the step of detecting in real-time and obtaining the present operating current specifically comprises:
collecting a feedback signal output by an ultrasonic driving module in real-time to obtain sampling data;
processing the sampling data to obtain electrical parameters;
the present operating current is obtained based on the electrical parameters.

6. A cutting control device applied to an ultrasonic knife, **characterized in** comprising:
a detection module, is used to detect a present active power of the ultrasonic knife;
an adjustment module, is used to gradually lower in a stepwise manner a rated operating current of the ultrasonic knife according to a predetermined time interval when the present active power is greater than a predetermined power critical value, and in the case that a duration of the present active power greater than the power critical value reaches a first predetermined time.

7. The device according to claim 6, **characterized in** further comprising a determination module used to determine the predetermined power critical value, the determination module is specifically used to:
determining an operating current range based on the rated operating current at an initial working moment of the ultrasonic knife;
detecting in real-time and obtaining a present operating current;
when the present operating current meets the operating current range, and in the case that a duration of the present operating current meeting the operating current range reaches a second predetermined time, detecting an active power at each moment within a predetermined time period;
determining the power critical value based on the active powers.

8. The device according to claim 6, **characterized in that**, the adjustment module is specially used to:
calculate and obtain a target rated operating current based on a predetermined current ratio and a present rated operating current, according to a predetermined time interval;
adjust the present rated operating current of the ultrasonic knife to the target rated operating current.

9. The method according to claim 8, **characterized in** further comprising a judgment module, the judgement module is specially used to judge whether the target rated current is less than the preset minimum rated current;
the judgment module is specially used to adjust the present rated operating current of the ultrasonic knife to the minimum rated current, in the case that the target rated current is less than the minimum rated current.

10. A storage medium, **characterized in that**, the storage medium stores a computer program, when it is executed by a processor, the computer program realizes the steps of the cutting control method applied to the ultrasonic knife of any of the above claims 1-5.
